# FASCICULE DE BREVET EUROPEEN

(11) **EP 0 950 426 B1**
(45) Date de publication et mention de la délivrance du brevet: **29.06.2005**
(21) Numéro de dépôt: 99400882.9
(22) Date de dépôt: 09.04.1999
(51) Int. Cl.: A61N 1/05

(54) **Sonde à impédance pour dispositif médical implanté, notamment pour stimulateur cardiaque**
Impedanzelektrode für eine implantierbare medizinische Vorrichtung, insbesondere für einen Herzschrittmacher
Impedance electrode for an implanted medical device, particularly for a heart pacemaker

(30) Priorité: 15.04.1998 FR 9804677
(43) Date de publication de la demande: 20.10.1999
(73) Titulaire: ELA MEDICAL (Société anonyme), 92541 Montrouge (FR)
(72) Inventeur: Guedeney, Dominique, 78470 Saint Remy de Chevreuse (FR); Bailly, Alain, 75014 Paris (FR)
(74) Mandataire: Dupuis-Latour, Dominique

(56) Documents cités:
- EP-A- 0 622 090
- EP-A- 0 791 372
- FR-A- 2 607 013
- GB-A- 2 096 001
- US-A- 5 300 107
- US-A- 5 443 492

## Description

L'invention concerne les sondes pour dispositifs médicaux implantés, notamment les sondes de stimulation cardiaque.

Typiquement, ces sondes comportent une gaine isolante creuse en matériau souple avec un conducteur électrique interne (deux conducteurs dans le cas d'une sonde bipolaire), terminée à son extrémité distale par une surface d'appui destinée à venir en contact avec l'endocarde et pourvue d'une électrode (électrode "distale" dans le cas d'une sonde bipolaire) permettant de réaliser la stimulation du myocarde.

L'électrode (distale) de stimulation présente généralement une extrémité frontale aplatie ou arrondie constituant la surface active proprement dite en contact avec la paroi du myocarde. Les EP-A-0 622 090 et EP-A-0 791 372 décrivent de telles sondes, où l'extrémité distale en contact avec le myocarde a la forme d'une boule conductrice émergeant d'un tube cylindrique isolant.

La conception d'une telle extrémité de sonde doit permettre de satisfaire divers impératifs, jusqu'à présent contradictoires.

La première exigence, essentielle, est la recherche d'une impédance élevée à l'interface coeur/électrode, afin de diminuer le courant nécessaire à la stimulation et, par voie de conséquence, accroître la durée de vie du stimulateur.

Pour accroître cette impédance, il est souhaitable de réduire la surface active de stimulation (voir notamment Clémenty et coll. "Économies d'énergie : le rôle de la sonde", Stimucoeur 1998, *26*, n° 4, pp. 184-187). Mais une réduction dimensionnelle de l'extrémité de la sonde entraîne une augmentation de la pression à l'interface coeurlélectrode conduisant à des élévations de seuil, voire des perforations.

Dans une géométrie particulière connue, l'électrode de stimulation s'étend sur une surface annulaire de l'extrémité frontale aplatie, dont la région centrale est isolante. On peut avoir une faible surface conductrice sans pour autant réduire la surface d'appui contre l'endocarde. Cette structure constitue une première solution de compromis entre les contraintes exposées ci-dessus.

Par ailleurs, le choix pour l'électrode d'un matériau tel qu'un carbone vitreux microporeux à la place d'un métal (platine) permet de combiner une excellente biocompatibilité avec des performances électriques satisfaisantes (notamment faibles pertes par polarisation).

Toutefois, même dans ce cas, l'impédance de contact reste néanmoins relativement faible, de l'ordre de 500 Ω. Par ailleurs, la forme annulaire de l'électrode conduit à des pertes de courant directement dans le sang, au travers de la partie de l'anneau qui n'est pas en contact avec le myocarde, ce qui constitue un facteur supplémentaire, préjudiciable à la durée de vie du stimulateur, de diminution de l'impédance de contact.

L'un des buts de l'invention est de proposer une structure de sonde qui permette de pallier ces divers inconvénients, en procurant notamment une impédance de contact élevée, pouvant aisément atteindre 1000 Ω, tout en maintenant une surface de contact optimale entre l'électrode et l'endocarde.

Mais on verra également que la structure particulière de l'invention permet de mettre à profit des phénomènes particuliers susceptibles d'améliorer encore l'efficacité de la stimulation, notamment les effets de pointe et la présence de gradients de potentiel élevés.

Ces avantages, on le verra également, sont obtenus avec une structure très peu traumatisante pour le patient, évitant donc ainsi tout risque de perforation du myocarde.

La sonde de l'invention est du type général décrit par le EP-A- 0 622 090 précité, qui comporte un corps cylindrique présentant côté distal une surface de contact avec l'endocarde, électriquement isolante, et une électrode de stimulation, électriquement conductrice, reliée à un conducteur de la sonde et qui comporte au moins un élément actif présentant à son extrémité libre une région faisant saillie par rapport à la surface de contact avec l'endocarde, cet élément actif présentant sur au moins une partie de son étendue en contact avec l'endocarde un rayon de courbure inférieur à 0,5 mm, avantageusement inférieur ou égal à 0,3 mm.

Selon l'invention, l'élément actif de la sonde de l'invention est un élément de forme plate s'étendant dans un plan radial de l'extrémité de sonde, en faisant saillie par rapport à la surface de contact avec l'endocarde le long d'une méridienne de celle-ci.

Le rayon de courbure peut alors être inférieur à 0,1 mm, de préférence inférieur à 0,02 mm, dans un plan perpendiculaire au plan de l'élément de forme plate.

Lorsque la surface de contact avec l'endocarde est hémisphérique, l'élément actif peut être en forme de disque plat. On peut par ailleurs prévoir deux éléments actifs de forme plate s'étendant dans deux plans radiaux orthogonaux de l'extrémité de sonde.

Le matériau de l'élément actif est de préférence un carbone vitreux microporeux.

D'autres caractéristiques et avantages de l'invention apparaîtront à la lecture de la description ci-dessous d'exemples de réalisation, en référence aux dessins annexés.

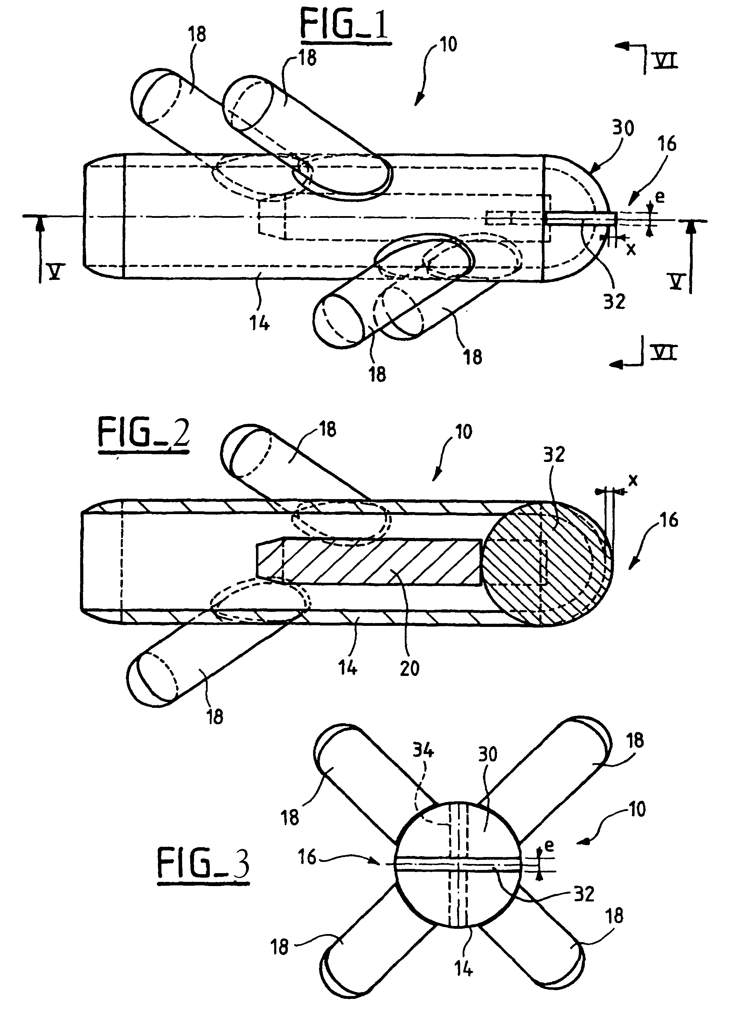

La figure 1 est une vue latérale d'une extrémité de sonde selon l'invention.
La figure 2 est une coupe longitudinale de cette même extrémité de sonde, prise selon V-V de la figure 1.
La figure 3 est une vue de bout de l'extrémité de sonde illustrée figure 1.

Sur les figures, la référence 10 désigne de façon générale l'extrémité distale d'une sonde de stimulateur cardiaque, qui comporte, à l'extrémité d'une gaine isolante flexible 12 enfermant un conducteur métallique (deux conducteurs dans le cas d'une sonde bipolaire), un corps isolant 14 de forme généralement cylindrique terminé par une surface destinée à venir en contact avec la paroi interne du myocarde et portant une électrode conductrice pour la stimulation (ainsi que, éventuellement, la détection).

Le corps cylindrique 14 et la gaine isolante 12 sont en un matériau électriquement isolant et par ailleurs biocompatible, généralement un élastomère de silicone ou de polyuréthanne. Une partie du corps 14 peut par ailleurs être chargée d'un principe actif tel qu'un stéroïde destiné à être diffusé dans la région du myocarde au voisinage de l'électrode afin d'améliorer les performances de celle-ci après implantation.

Le corps est généralement pourvu de barbes d'ancrage 18 destinées à venir s'imbriquer dans les trabécules cardiaques pour permettre le maintien en position de l'extrémité de la sonde après son implantation. Dans d'autres réalisations, on peut prévoir un système d'ancrage à vis, comme par exemple celui de la sonde modèle US46 d'ELA Médical.

Le corps 14 porte l'électrode de stimulation 16, typiquement en carbone vitreux microporeux, reliée côté proximal au conducteur métallique interne (non représenté) par une tige axiale 20.

Côté distal, l'électrode de stimulation16 est formée d'un disque de faible épaisseur, s'étendant dans un plan radial sensiblement suivant une demi-circonférence de la surface de contact hémisphérique 30, avec un léger décalage en saillie par rapport à cette dernière.

Le disque 32 est un disque en matériau conducteur, notamment en carbone vitreux microporeux, qui est relié électriquement et mécaniquement à la tige axiale 20, par exemple par soudure à celle-ci.

De préférence, et de manière notamment à respecter les tolérances dimensionnelles qui seront indiquées plus bas, le disque 32 est inséré dans l'extrémité hémisphérique distale de la sonde après moulage du matériau silicone du corps 16.

Dans une réalisation particulière, le diamètre de la surface de contact 30 est de 2 mm, et le disque présente un diamètre de 2 mm également, pour une épaisseur e de 0,2 mm et une saillie x de 0,1 mm.

Cette géométrie procure les avantages suivants :
― extrémité de sonde peu traumatisante pour le muscle cardiaque,
― contact intime maximal avec la surface de l'endocarde, concourant à une meilleure circulation,
― faible rayon de courbure des parties saillantes, permettant d'obtenir une extrémité de sonde peu traumatisante,
― gradient de potentiel important et effet de pointe contribuant à une amélioration du phénomène de stimulation.

À cet égard, l'effet de pointe se localise principalement sur les arêtes semi-circulaires du disque 32 dans la région apparente de celui-ci. Un usinage du disque en carbone permet de réaliser sans difficulté ces arêtes avec un rayon de courbure (considéré dans un plan perpendiculaire au plan du disque) de l'ordre de 0,01 mm, permettant de produire un effet de pointe marqué, et ceci sur toute la demi-circonférence apparente du disque.

Dans une variante de mise en oeuvre, au lieu d'un unique disque 32 s'étendant dans un plan radial de la sonde, on peut prévoir deux disques disposés en croix 32, 34 (figure 6), s'étendant dans deux plans radiaux orthogonaux de la sonde.

## Revendications

1. Une sonde pour dispositif médical implanté, notamment pour stimulateur cardiaque, comportant
un corps cylindrique (14) présentant côté distal une surface (30) de contact avec l'endocarde, électriquement isolante, et
une électrode de stimulation (16) électriquement conductrice, reliée à un conducteur de la sonde et comportant au moins un élément actif (32) présentant à son extrémité libre une région faisant saillie par rapport à la surface de contact avec l'endocarde, cet élément actif présentant sur au moins une partie de son étendue en contact avec l'endocarde un rayon de courbure inférieur à 0,5 mm, avantageusement inférieur ou égal à 0,3 mm, sonde **caractérisée en ce que** l'élément actif est un élément de forme plate s'étendant dans un plan radial de l'extrémité de sonde, en faisant saillie par rapport à la surface de contact avec l'endocarde le long d'une méridienne de celle-ci.

2. La sonde de la revendication 1, dans laquelle le matériau de l'élément actif est un carbone vitreux microporeux.

3. La sonde de la revendication 1, dans laquelle ledit rayon de courbure est inférieur à 0,1 mm, de préférence inférieur à 0,02 mm, dans un plan perpendiculaire au plan de l'élément de forme plate.

4. La sonde de la revendication 1, dans laquelle la surface de contact avec l'endocarde est hémisphérique et l'élément actif est en forme de disque plat.

5. La sonde de la revendication 1, comportant deux éléments actifs de forme plate s'étendant dans deux plans radiaux orthogonaux de l'extrémité de sonde.

## Patentansprüche

1. Sonde für eine implantierte medizinische Vorrichtung, insbesondere einen Herzschrittmacher, aufweisend: einen zylindrischen Körper (14), der auf einer distalen Seite eine Oberfläche (30) des Kontakts mit dem Endokard aufweist, die elektrisch isolierend ist, und eine Stimulationselektrode (16), die elektrisch leitend ist, verbunden mit einem Leiter der Sonde und wenigstens ein aktives Element (32) aufweisend, das an seinem freien Ende einen im Verhältnis zur Oberfläche des Kontakts mit dem Endokard hervorstehenden Bereich aufweist, wobei das aktive Element wenigstens auf einen Teil seiner Ausdehnung im Kontakt mit dem Endokard einen Biegeradius von weniger als 0,5 mm, vorteilhafterweise kleiner oder gleich 0,3 mm aufweist, wobei die Sonde **dadurch gekennzeichnet ist, dass** das aktive Element eine flache Form aufweist, die sich in einer radialen Ebene des Endes der Sonde erstreckt, während sie im Verhältnis zur Oberfläche des Kontakts mit dem Endokard entlang einer Mittellinie derselben hervorsteht.

2. Sonde nach Anspruch 1, bei der das Material des aktiven Elements ein glasartiger mikroporöser Kohlenstoff ist.

3. Sonde nach Anspruch 1, bei welcher der Krümmungsradius geringer als 0,1 mm ist, vorzugsweise geringer als 0,02 mm, in einer Ebene senkrecht zur Ebene des Elementes von flacher Form.

4. Sonde nach Anspruch 1, bei welcher die Oberfläche des Kontakts mit dem Endokard halbkugelförmig ist und das aktive Element die Form einer flachen Scheibe aufweist.

5. Sonde nach Anspruch 1, welche zwei aktive plattenförmige Elemente aufweist, die sich in zwei radialen orthogonalen Ebenen des Endes der Sonde erstrecken.

## Claims

1. A lead for an implantable medical device, in particular a cardiac pacemaker, comprising:
a cylindrical body (14) having on its distal side an electrically insulated surface (30) for contacting the endocardium, and
an electrically conducting stimulation electrode (16) connected to a conductor of the lead and comprising at least one active element (32) having at its free end an area protruding relative to the surface contacting the endocardium, said active element having on at least part of its area contacting the endocardium a radius of curvature less than .5 mm, preferably less than or equal to .3 mm,
said lead being **characterised in that** the active element is an element having a flat form extending in a radial plane of the tip of the lead, while protruding from the surface contacting the endocardium along a meridian thereof.

2. The lead of claim 1, wherein the material of the active element is a microporous vitreous carbon.

3. The lead of claim 1, wherein said radius of curvature is less than .1 mm, preferably less than .02 mm, in a plane orthogonal to the plane of the element having a flat form.

4. The lead of claim 1, wherein the surface contacting the endocardium is semi-spherical and the active element is in the form of a flat disc.

5. The lead of claim 1, comprising two active elements having a flat form which extend in two orthogonal planes of the tip of the lead.
